(19) Europäisches Patentamt / European Patent Office / Office européen des brevets

(11) **EP 3 108 869 B1**

(12) **EUROPEAN PATENT SPECIFICATION**

(45) Date of publication and mention
of the grant of the patent:
**08.04.2020 Bulletin 2020/15**

(21) Application number: **15752638.5**

(22) Date of filing: **05.02.2015**

(51) Int Cl.:
*A61K 8/19* (2006.01)          *A61K 8/27* (2006.01)
*A61K 8/29* (2006.01)          *A61K 8/34* (2006.01)
*A61K 8/894* (2006.01)         *A61Q 17/04* (2006.01)
*A61K 8/25* (2006.01)          *A61K 8/26* (2006.01)
*A61K 8/28* (2006.01)          *A61K 8/02* (2006.01)

(86) International application number:
**PCT/JP2015/053244**

(87) International publication number:
**WO 2015/125622 (27.08.2015 Gazette 2015/34)**

(54) **AQUEOUS DISPERSION OF INORGANIC POWDER PARTICLES SUBJECTED TO HYDROPHOBIC ORGANIC SURFACE TREATMENT, AND COSMETIC INCLUDING SAME**

WÄSSRIGE DISPERSION VON ANORGANISCHEN PULVERPARTIKELN, DIE EINER HYDROPHOBEN ORGANISCHEN OBERFLÄCHENBEHANDLUNG UNTERZOGEN WURDEN, UND KOSMETIK DAMIT

DISPERSION AQUEUSE DE PARTICULES DE POUDRE INORGANIQUE SOUMISES À UN TRAITEMENT DE SURFACE ORGANIQUE HYDROPHOBE, ET PRODUIT COSMÉTIQUE LA CONTENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.02.2014 JP 2014030479**

(43) Date of publication of application:
**28.12.2016 Bulletin 2016/52**

(73) Proprietor: **Sakai Chemical Industry Co., Ltd.**
**Sakai, Osaka 590-8502 (JP)**

(72) Inventors:
• **ASHIDA, Takuro**
**Sakai-shi**
**Osaka 590-0985 (JP)**
• **MAGARA, Koichiro**
**Iwaki-shi**
**Fukushima 971-8183 (JP)**

• **SANO, Akifumi**
**Iwaki-shi**
**Fukushima 971-8183 (JP)**
• **SHIKE, Ayana**
**Iwaki-shi**
**Fukushima 971-8183 (JP)**

(74) Representative: **Isarpatent**
**Patent- und Rechtsanwälte Behnisch Barth Charles**
**Hassa Peckmann & Partner mbB**
**Friedrichstrasse 31**
**80801 München (DE)**

(56) References cited:
EP-A1- 2 740 774      EP-A2- 1 772 138
WO-A1-2013/018827     JP-A- H06 116 120
JP-A- H07 247 119     JP-A- 2000 072 623
JP-A- 2007 119 741    JP-A- 2010 143 852
US-A1- 2011 129 509

EP 3 108 869 B1

**Description**

TECHNICAL FIELD

The present invention relates to a water-based dispersion which contains an inorganic powder microfine particle subjected to a hydrophobic organic surface treatment, a polyhydric alcohol, water, and a nonionic surfactant, wherein an amount of the inorganic powder microfine particle is 30 wt% or more, as defined in claim 1.

BACKGROUND OF THE DISCLOSURE

Recently, a suncare product has attracted attention because of raising public consciousness of ultraviolet. The product contains an organic UV filter and/or an inorganic UV filter. The inorganic UV filter is desired strongly because of safety, so suncut cosmetics containing inorganic powder microfine particles such as titanium oxide, zinc oxide, and cerium oxide as a sunscreen agent are receiving a lot of attention.

Generally, a dispersion prepared by dispersing the inorganic powder microfine particles in an oil medium is known, this dispersion is used for pruducts such as a W/O type sunscreen cream and lotion. However, the touch feeling of the W/O type cosmetic is inferior and the W/O type cosmetic is hard-to-remove, so that an O/W type suncut cosmetic is paid attention. Therefore, a water-based dispersion of inorganic powder microfine particles being available in the O/W type products puts on the market. However, when such dispersion is formulated in the O/W type product, there is a problem described later and the effect thereof can not be expressed efficiently.

In many of the O/W products, the aqueous phase thereof contains a water-soluble high polymer and the stability is maintained by the structural viscosity of the water-soluble high polymer. A hydration part of the water-soluble high polymer may be carboxylic acid type or sulfonic acid type. Anyway, an anionic group is contained in the high polymer structure.

The inorganic powder microfine particles are needed to be used stably without occurring a viscosity reduction and an aggregation with the anionic hydration high molecular. Specifically, when the carboxylic acid type water-soluble high polymer is used, the following conditions are needed to be satisfied;

An elution of a polyvalent metal from dispersed particles will not be occurred, and
the inorganic powder or inorganic powder of which surface is primary treated have no aggregability with the water-soluble high polymer which is hydrous swollen.

Many of water dispersions of titanium oxide being on sale now block the surface activity of titanium oxide microfine particles by primary treating the titanium oxide microfine particles with silica, and utilize the hydration ability of a silanolgroup contained in silica by surface treating for improving the hydrophilicity. However, about the titanium oxide treated with silica, the elution of polyvalent metal is reduced, on the otherhand, the problem that the water-soluble high polymer occurs the viscosity increasing with a silanol group. Further, when a large amount of the dispersion is used, the viscosity increasing and gelling of the O/W products are occurred with time, so that the products sometimes become unusable. A creaking feeling of the product on the skin derived from strong aggregability of the silanol group is occurred to deteriorate the touch feeling. Further, the water resistance for sweat, water, and so on becomes inferior.

As the prior technique about water dispersions of microfine particle powder, patent documents 1 and 2 are disclosed.

Patent document 1 discloses a water-based dispersion of titanium oxide microfine particles in consideration of an electrolyte (for example, sodium chloride and potassium chloride). The electrolyte is rarely used in a water-soluble high polymer system providing the structural viscosity which is the main system in the present O/W formulations, and a water-soluble high polymer providing the structural viscosity is not used in examples. The products in examples are high-viscosity products using monoglycerides, or higher alcohols, and not low-viscosity O/W type produsts using a wate-soluble high polymer. Further, the production method of patent document 1 comprises a step of dissolveing a dispersant in water so that the method is different from the present disclosure. It is disclosed that titanium oxide is primary treated with aluminium, silicon, zirconium, zinc, or tin. However, aluminium is generally aluminium hydroxide, and zinc is an amphiprotic compound. Therefore, when treated with these compounds, an elution of aluminium metal and zinc metal is occurred so that a water dispersion of titanium oxide may be formed but a stability thereof can not be maintained in a water-soluble high polymer system which is the main type presently.

Patent document 2 relates to a water-based dispersion technique, and does not disclose about the stability of O/W type products which is the main type presently. The importance of the polyhydric alcohol is disclosed only as an antiseptic agent. It is difficult to prepare a water dispersion with a powder amount of 20 wt % or more considering examples.

Therefore, a water dispersion containing high concertation of inorganic powder microfine particles is recommended, and a water-based dispersion containing the same which can be compounded stably in an anionic water-soluble high polymer system O/W product without the viscosity reduction of the O/W product or gelation by the aggregation, and which is superior in feeling without a creaking feeling and in the water resistnace for sweat and water is recommended.

PRIOR TECHNICAL DOCUMENTS

PATENT DOCUMENTS

[Patent Document 1] Japanese Patent No.2852487
[Patent Document 2] WO2013/018827

SUMMARY OF INVENTION

PROBLEM TO BE SOLVED BY THE INVENTION

It is one of the objects of the present disclosure to provide a water-based dispersion containing inorganic powder microfine particles subjected to a hydrophobic organic surface treatment of 30 wt% or more, and containing a polyhydric alcohol, water and a nonionic surfactant, as defined in claim 1, and to prepare a stable O/W product having an ultralight shielding effect, and to provide a cosmetic having a good feeling and a strong water repellency for sweat and water, by blending the water-based dispersion to a cosmetic.

MEANS FOR SOLVING OBJECT

The inventors could prepare a dispersion in which inorganic powder microfine particles subjected to a hydrophobic organic surface treatment may be dispersed uniformly into a water system containing a polyhydric alcohol and water by selecting a specified nonionic surfactant. The dispersion may contain the inorganic powder microfine particles of 30 wt% or more, and, when contained in an O/W product, a product having a good touch feeling and a high water repellency may be prepared without occurring problems such as viscosity reduction and gelation to complete the present disclosure.

The present disclosure relates to a water-based dispersion which contains an inorganic powder microfine particle subjected to a hydrophobic organic surface treatment, a polyhydric alcohol, water, and a nonionic surfactant, wherein the nonionic surfactant is a compound which can be dissolved transparently or slightly muddy in 1,3-butylene glycol (20 wt% concentration, 35°C), and an amount of the inorganic powder microfine particle is 30 wt% or more, as defined in claim 1.

The inorganic powder microfine particle is preferably at least one selected from the group consisting of titanium oxide, zinc oxide, and cerium oxide.

A part surface or all surface of the inorganic powder microfine particle is preferably covered by at least one selected from the group consisting of silica, aluminium hydroxide, alumina, and zirconia.

The polyhydric alcohol is preferably one or more polyhydric alcohol selected from the group consisting of propylene glycol, butylene glycol, pentane diol, dipropylene glycol, hexanediol, and heptanediol.

The nonionic surfactant is preferably insoluble or dispersed cloudy in water (20 wt% concentration, 35°C).

The nonionic surfactant is preferably an organopolysiloxane nonionic surfactant.

The present disclosure relates to a cosmetic obtained by mixing the water-based dispersion.

EFFECT OF THE INVENTION

The present disclosure relates to a water dispersion in which inorganic powder microfine particles subjected to a hydrophobic organic surface treatment of 30 wt% or more may be dispersed uniformly in a water system containing the inorganic powder microfine particles, a polyhydric alcohol, and water by using a specific nonionic surfactant. Further, a cosmetic obtained by blending the water dispersion in an O/W product of an anionic water-soluble high polymer is superior in quality without viscosity reduction and gelation of the product, and has a good touch feeling without creaking feeling and high water repellency, and is superior in usability.

DETAILED DESCRIPTION OF PREFERRED EMBODIMENTS

A primary particle diameter of inorganic powder microfine particles to be used in the present disclosure is preferably an average particle diameter of 5 to 200 nm which can generally show UV absorption/scattering effect. The

3

lower limit is more preferably 10 nm, and the upper limit is more preferably 100 nm. In the specification, the particle diameter is measured by measuring the particle diameters of randomly selected 200 particles with an electron microscope, and calculating the average particle diameter of the primary particles.

The inorganic powder microfine particle is not particularly limited but may include titanium oxide, zinc oxide, and cerium oxide, and two or more kinds of them may be used in combination and composites thereof may be used.

As the shape of the inorganic powder microfine particle, a powder having any shape can be used, including spherical, rod-shaped, needle-shaped, spindle-shaped, plate-shaped, and hexagonal plate-shaped particles, and needle-shaped aggregates. In rod-shaped, needle-shaped, or spindle-shaped particles, the average particle diameter is defined as the length in the direction of the short axis. In plate-shaped particles, the average particle diameter is defined as the average diameter of the maximum inscribed circle of the plane.

The inorganic powder microfine particles are preferably obtained by hydrophobic organic surface treating a composite powder which is obtained by covering with another inorganic material to block the surface activity of titanium oxide, zinc oxide, or cerium oxide.

The inorganic material for the surface treatment may be one or more of silica, aluminium hydroxide, alumina, and zirconia. A covering amount is preferably 1 to 25 wt% relative to the total amount of the inorganic powder, and more preferably 2 to 20 wt%. When a compound such as aluminium hydroxide which dissociates in water and generates a polyvalent metal ion is used, an aluminium ion is eluted in water system to reduce the viscosity of the anionic water-soluble high polymer. Therefore, an amount is limited, and preferably 10 wt% or less. Further, from these viewpoints, a compound such as silica without causing an ion elution is preferred.

A hydrophobic organic surface treatment agent for treating the inorganic powder microfine particle includes, for example, methylhydrogen polysiloxanes, dimethyl polysiloxanes, copolymers of methylhydrogen polysiloxane and dimethyl polysiloxane, dimethyl polysiloxanes containing a reactive trialkoxysilyl group such as a trimethoxysilyl group and a triethoxysilyl group, alkyl-modified polysiloxanes containing the same reactive group, dimethyl polysiloxanes, alkylsilanes, alkyl titanates, and metal soaps. The inorganic powder microfine particle may be treated by a known method using at least one selected from the above-mentioned compunds. By treating with the hydrophobic organic surface treating agent, the hydrophilic groups of the inorganic material for the surface treatment are blocked and the gel formation of the inorganic powder with the water-soluble high polymer is suppressed so that the creaking feeling may be prevented at the time of use and water resistance may be improved.

In the hydrophobic organic treatment of the inorganic powder, from 2 to 15 wt% of the total amount of the inorganic powder after treatment is preferably subjected to the organic treatment. If the amount of the inorganic powder subjected to the organic treatment is less than 2 wt%, it is undesirable in view of insufficient hydrophobicity. If the amount is more than 15 wt%, it is undesirable because the effect of hydrophobicity is saturated. More preferably, from 4 to 12 wt% of the total amount of the inorganic powder is subjected to the organic treatment.

The water-based dispersion contains the inorganic powder of 30 wt% or more relative to the total amount of the dispersion. The amout is preferably within the above-mentioned range because a small amount of the dispersion can express an effect as a cosmetic material. The amount is more preferably 40 wt% or more. The upper limit of the amount is not particularly limited but is preferably 70 wt% or less, and more preferably 60 wt% or less.

The water-based dispersion of the present disclosure contains a polyhydric alcohol. The polyhydric alcohol may be one or more polyhydric alcohol selected from the group consisting of propylene glycol, butylene glycol, pentane diol, dipropylene glycol, hexanediol, and heptanediol, and butylene glycol, pentane diol, dipropylene glycol, and hexanediol are especially preferred. An amount of the polyhydric alcohol is 5 to 25 wt% in the water-based dispersion. The lower limit thereof is more preferably 8 wt%, and the upper limit thereof is more preferably 20 wt%. The nonionic surfactant can be uniformly orientated on the powder surface to stabilize the dispersion by blending the polyhydric alcohol.

The water-based dispersion of the present disclosure contains a nonionic surfactant. The nonionic surfactant may be used singly or two or more of them may be used in combination. When the nonionic surfactant is mixed with 1,3-butylene glycol with a concentration of 20 wt%, the nonionic surfactant is dissolved transparently or slightly muddy at 35°C (when the nonionic surfactant is paste form or solid at room temperature, the condition is confirmed at 35°C after heating to make uniform) . In the case of using two or more kinds of nonionic surfactants in combination, a mixture of two or more nonionic surfactants obtained by mixing them at the same ratio as the mixing ratio in the water-based dispersion is necessarily dissolved transparently or slightly muddy when the above-mentioned test is carried out.

In the specification, "dissolved transparently" means that a haze value of the obtained mixture at an optical path length of 10 mm and 35°C is less than 10. "Dissolved slightly muddy" means that a haze value of the obtained mixture at an optical path length of 10 mm and 35°C is 10 or more, and the total light transmittance is 30% or more.

By using the nonionic surfactant, a layer of the nonionic surfactant can be formed on the particle surface efficiently. An amount of the nonionic surfactant is preferably 1 to 25 wt% relative to the total amount of the dispersion. The lower limit is more preferably 2 wt%, and still more preferabnly 3 wt%. The upper limit is more preferably 20 wt%, more preferably 15 wt%.

When the nonionic surfactant is mixed with water with a concentration of 20 wt%, the surfactant is insoluble or dispersed cloudy (when the nonionic surfactant is paste form or solid at room temperature, the condition is confirmed at 35°C after heating to make uniform). In the case of using two or more kinds of nonionic surfactants in combination, a mixture of two or more nonionic surfactants obtained by mixing them at the same ratio as the mixing ratio in the water-based dispersion is necessarily insoluble or dispersed cloudy when the above-mentioned test is carried out.

In the specification, "insoluble" means that an unmelted residue is caused in the mixture, or a phase separation is occurred an hour later even if the mixture looks muddy. "Dispersed cloudy" means that a haze value of the obtained mixture at an optical path length of 10 mm and 35°C is 10 or more, and the total light transmittance is less than 30%.

An HLB value of the surfactant is 6 to 12. When a dispersion which is obtained by dispersing with the use of only the nonionic surfactant having an HLB value of over 12 is blended in an O/W product, the solubility of the nonionic surfactant to water is too high so that the adsorption ability to the powder becomes ungood. Further, the viscosity increasing of the product tends to occur and the water resistance tends to deteriorate. When the HLB value of the nonionic surfactant is less than 6, a water dispersion cannot be prepared. In the specification, the HLB value is calculated by the following expression as defined by W. C. Grifinn:

$$N_{HLB} = (E + P)/5$$

($N_{HLB}$: HLB value, E: wt% of a polyoxyethylene moiety based on the whole molecules of the dispersant, P: wt% of a polyhydric alcohol moiety based on the whole molecules of the dispersant).

The nonionic surfactant include one or more compounds selected from a polyoxyalkylene fatty acid ester, a polyoxyalkylene sorbitan fatty acid ester, and an organopolysiloxane having a polyoxyalkylene group.

Further, the organopolysiloxane nonionic surfactant as mentioned above is most preferred. When the organopolysiloxane nonionic surfactant is used, the especially excellent effects such as a good stability of a composition and a good feeling in use as a cosmetic can be given.

A dispersing method for preparing the water-based dispersion of the present disclosure may include known methods such as a method using a bead mill, a jet mill or a high-pressure homogenizer which can disperse uniformly.

The water-based dispersion of the present disclosure may contain an antiseptic preservative and an antibacterial agent such as alkyl ester of paraoxybenzoic acid, benzoic acid, sodium benzoate, sorbic acid, potassium sorbate, phenoxyethanol, salicylic acid, lime acid, sorbic acid, p-chlorometacresol, hexachlorophen, benzalkonium chloride, chlorhexidine chloride, photosentive element, and phenoxyethanol, unless the performance of the dispersion is deteriorated.

The water-based dispersion of the inorganic powder microfine particles subjected to a hydrophobic organic surface treatment thus obtained is useful as a component for a cosmetic having an ultraviolet shielding effect. In this case, only the water-based dispersion of the present disclosure may be used as an ultraviolet shielding component, but an oil-based dispersion of inorganic powder microfine particles or an organic ultraviolet absortion agent may be used in combination.

In another aspect, the present invention relates to the use of the water-based dispersion to obtain a cosmetic. A production method of the cosmetic is not particularly limited, and the cosmetic is obtained by a normal production mehod, by blending the water-based dispersion in a cosmetic.

The cosmetic is not particularly limited, and the water dispersion may be blended in cosmetics which are used externally for skin and hair such as skin care products, hair care products, make up products, ultraviolet protection products. A form of the cosmetic is not particularly limited but may be a liquid form, an emulsion form, a cream form, a solid form, a paste form, a gel form, a multilayer form, a mousse from, a spray form, and so on.

The cosmetic of the present disclosure is especially suitably used in a system in which an anionic water-soluble high polymer is used in combination related to O/W cosmetics obtained by using a water soluble high polymer in a water phase. That is, the advantage that the viscosity reduction and gelation by combined with an anionic water-soluble high polymer may not occur can be given. As such anionic water-soluble high polymer, for example, carboxyvinyl polymer may be used.

EXAMPLES

While the present invention will now be described in more detail with reference to the examples, the present invention is not limited to these examples. Unless otherwise noted, in the examples and comparative examples, "%" means wt%.

(Examples 1 to 10, and comparative examples 1 to 2)

The examples 2, 4, 7 and 10 are not according to the invention as claimed. The nonionic surfactant shown in table 1 10.0g and silicone surface-treated, silica surface-treated titanium oxide microfine particles (STR-100W-LPT: manufactured by Sakai Chemical Industry Co., Ltd., spindle-shaped particles having a particle diameter of 20 nm in the direction of the short axis and 100 nm in the direction of the long axis) 40.0 g were mixed in 1,3 butylene glycol 10.0 g and then water 40 g was added. Next, the mixture was stirred and it was confirmed whether or not the water dispersion of titanium oxide could be prepared. The solubility of the nonionic surfactant in 1,3-butylene glycol or water at 35°C was confirmed by mixing the used nonionic surfactant 2.0 g in 1,3-butylene glycol 8.0 g or water 8.0 g, respectively. The HLB of each nonionic surfactant was confirmed. The results are shown in table 2.

(Comparative example 3)

A water dispersion of titanium oxide was prepared by the same procedure except that titanium oxide microfine particles not hydrophobic organic surface treated are used.

(Examples 11 to 20, and comparative example 4)

Sunscreen O/W creams of examples 11 to 20, and comparative example 4 having the composition as shown below were prepared by using the water dispersions of titanium oxide obtained in examples 1 to 10 and comparative example 3, and the qualities were evaluated

Table 1

(Sunscreen O/W cream)

| (Components) | | (%) |
|---|---|---|
| 1 | Hydrogenated polyisobutene | 21.0 |
| 2 | Behenyl alcohol | 1.0 |
| 3 | Sorbitan sesquioleate | 0.5 |
| 4 | Glyceryl stearate (SE) | 0.8 |
| 5 | Carbomer | 0.1 |
| 6 | Acrylate/alkyl acrylate (C10-C30) crosspolymer | 0.1 |
| 7 | Polysorbate 80 | 1.5 |
| 8 | Triethanolamine | 0.2 |
| 9 | 1,3-butylene glycol | 8.0 |
| 10 | Water dispersion of titanium oxide (examples 1 to 10, comparative example 3) | 25.0 |
| 11 | Purified water | 41.8 |

(Production method)

A: The components 1-4 are mixed and heated.
B: The components 5-11 are mixed uniformly and heated.
C: A is added to B to emulsify and then cooled to obtain each sunscreen O/W cream (examples 11 to 20 and comparative example 4) .

The evaluations were done according to the following method.

1. Preparation of water dispersion of titanium oxide

○: manufacturable
×: unmanufactured

2. Stability of sunscreen O/W cream

After the prepared O/W cream was left to stand at 50°C for a month, it was confirmed whether or not the cream was thickened.

[Evaluation criteria]

◎: not thickening
○: slightly thickening
△: thickening
✕: remarkably thickening and gelling

3. Evaluation of feeling in use

After a use test was done by 20 specialized panels for evaluation, the spreading and elongation at coating, the creaking feeling of skin after coating, and the sunscreen effect were evaluated on a scale of one to five based on the following criteria and then the average scores were determined to decide.

5 points: excellent good
4 points: good
3 points: normal
2 points: slightly ungood
1 point: ungood

[Decision]

◎: average score of 4.5 or more
○: average score of 3.5 or more and less than 4.5
△: average score of 2.5 or more and less than 3.5
✕: average score of less than 2.5

4. Total evaluation

◎: especially excellent in stability and feeling in use
○: excellent in stability and feeling in use
△: viscosity increasing is occurred or feeling in use is inferior
✕: stability is ungood and feeling in use is inferior

Table 2

| | Ex. 1 | Ex. 2 | Ex. 3 | Ex. 4 | Ex. 5 | Ex. 6 | Ex. 7 |
|---|---|---|---|---|---|---|---|
| Nonionic surfactant (ingredients) | NIKKOL MYS-10V[1] | NIKKOL MYS-25Y[1] | EMALEX PEIS-12EX[2] | EMALEX PEIS-20EX[2] | NIKKOL TO-30V[1] | NIKKOL TO-10V[1] | KF-6011[3] |
| Labeling name of ingredient | PEG-10 stearate | PEG-25 stearate | PEG-12 isostearate | PEG-20 isostearate | Polyoxyethylene Sorbitan Trioleate (20E.O.) | Polyoxyethylene Sorbitan monooleate | PEG-11methyl ether dimethicone |
| HLB | 11 | 15 | 12 | 14 | 11 | 15 | 14.5 |
| Solubility in 1,3-butylene glycol | Slightly muddy | Slightly muddy | Slightly muddy | Slightly muddy | Slightly muddy | Slightly muddy | Slightly muddy |
| Solubility in water | Cloudy | Transparently | Cloudy | Transparently | Cloudy | Transparently | Transparently |
| Preparation of water dispersion | ○ | ○ | ○ | ○ | ○ | ○ | ○ |
| | Ex. 11 | Ex. 12 | Ex. 13 | Ex. 14 | Ex. 15 | Ex. 16 | Ex. 17 |
| Stability | ○ | △ | ○ | △ | ○ | △ | △ |
| Feeling in use | ◎ | △ | ◎ | △ | ◎ | △ | △ |
| Total evaluation | ○ | △ | ○ | △ | ○ | △ | △ |

| | Ex. 8 | Ex. 9 | Ex. 10 | Compar. Ex. 1 | Compar. Ex. 2 | Compar. Ex. 3 |
|---|---|---|---|---|---|---|
| Nonionic surfactant (ingredients) | KF-6013[3] | KF-6017[3]:6043[3]=4:1 | KF-6043[3] | NIKKOL EGMS-70V[1] | EMALEX PEIS-3EX[2] | NIKKOL MYS-10V[1] |
| Labeling name of ingredient | PEG-9 dimethicone | PEG-10 dimethicone | PEG-10 dimethicone | Glycol stearate | PEG-12 isostearate | PEG-10 stearate |
| HLB | 10 | 6.5 | 14.5 | 3.5 | 7 | 11 |
| Solubility in 1,3-butylene glycol | Slightly muddy | Slightly muddy | Transparently | Insoluble | Insoluble | Slightly muddy |
| Solubility in water | Cloudy | Insoluble | Transparently | Insoluble | Insoluble | Cloudy |
| Preparation of water dispersion | ○ | ○ | ○ | × | × | ○ |
| | Ex. 18 | Ex. 19 | Ex. 20 | | | Compar. Ex. 4 |
| Stability | ◎ | ◎ | △ | | | × |
| Feeling in use | ◎ | ◎ | △ | | | × |
| Total evaluation | ◎ | ◎ | △ | | | × |

1) manufactured by Nikko Chemicals Co., Ltd.
2) manufactured by Nihon Emulsion Co., Ltd.
3) manufactured by Shin-Etsu Chemical Co., Ltd.

Concerning the used nonionic surfactant, a haze and a total light transmittance when mixed with 1,3-butylene glycol were measured. The measurement was done by putting each 20 wt% sample liquid at 35 °C in a cell at an optical path length of 10 mm and measuring using a haze meter (NDH4000 manufactured by Nippon Densyoku Industries Co.,

Ltd.). The results were shown in table 3.

Table 3

| Medium | Water | 1,3-butylene glycol | 1,3-butylene glycol | 1,3-butylene glycol | 1,3-butylene glycol | Water |
|---|---|---|---|---|---|---|
| Nonionic surfactant | - | - | KF-6043 | NIKKOL TO-10V | KF-6013 | KF-6013 |
| Haze | 0.1 | 0.20 | 2.84 | 99.37 | 80.82 | 99.48 |
| Total light transmittance (%) | 92 | 92 | 90 | 44 | 92 | 16 |
| Condition | - | - | Transparently | Slightly muddy | Slightly muddy | Cloudy |

As is evident from the results of tables 2 and 3, in the examples 1 to 10 of the present disclosure in which the nonionic surfactants which can be dissolved transparently or slightly muddy in 1,3-butylene glycol are used, low-viscosity water dispersions of titanium oxide which are superior in dispersibility can be prepared, even though the used nonionic surfactant may be insoluble or dispersed cloudy in water. On the other hand, in the comparative examples 1 to2 in which the nonionic surfactant which are insoluble in 1,3-butylene glycol, a water dispersion can not be prepared. Further, in the comparative example 3 in which titanium oxide which is not subjected to a hydrophobic organic surface treatment, high-viscosity water dispersion could be prepared.

Concerning the sunscreen O/W creams of the examples 11 to 20 and the comparative example 4 obtained by using the titanium oxide dispersions of the examples 1 to 10 and the comparative example 3, the O/W cream of the examples 11, 13, 15, 18, and 19 are stable without thickening over time. Furthermore, concerning the feeling in use thereof, the elongation and spreading at coating are excellent and creaking feeling of skin after coating is not occurred, and the obtained cream is found to be a O/W cream having an extremely high sunscreen effect. Among them, in the examples 18 and 19, the especially excellent O/W creams could be obtained. On the otherhand, the viscosity of the O/W creams of the examples 12, 14, 16, 17, and 20, respectively tends to increase, the elongation and the spreading at coating is good but the creaking feeling of skin after coating is occurred, and the sunscreen effect thereof are slightly inferior. Further, the O/W cream of the comparative example 4 was thickened remarkably over time to form a gel and the feeling in use was extremely ungood.

Considering from an overall perspective, the O/W creams obtained by using the water dispersions of titanium oxide prepared by using the nonionic surfactant of the examples 1, 3, 5, 8, and 9 are superior in the stability and the feeling in use, but the O/W creams obtained by using the water dispersions of titanium oxide prepared by using the nonionic surfactant of the examples 2, 4, 6, 7, and 10 have a disadvantage such as a viscosity increasing or are inferior in the feeling in use because the creaking feeling is occurred. Further, the O/W cream obtain by using the water dispersion of titanium oxide of comparative example 3 in which titanium oxide not subjected to hydrophobic organic surface treatment was dispersed has a bad stability and extremely inferior feeling in use.

Therefore, the water dispersion of titanium oxide microfine particles subjected to hydrophobic oraganic surface treatment which is obtained by using the nonionic surfactant dissolved transparently or slightly muddy in 1,3-butylene glycol and insoluble or dispersed cloudy in water is low-viscosity water dispersion having an excellent dispersibility. Therefore, the O/W cream containing this water dispersion is superior in the stability and the feelin in use. As shown especially in the examples 8 and 9, the temperature stability is improved by using the organopolysiloxane dispersant.

[Example 21]

DPG 10.0 g was put into BY25-339 (manufactured by Dow Corning Toray Co., Ltd.: PEG/PPG-30/10 dimethicone, HLB: 8.5, DPG) 20.0 g and then silicone surface-treated, silica surface-treated titanium oxide microfine particles (STR-100W-LPT manufactured by Sakai Chemical Industry Co., Ltd.: spindle-shaped particles having a short axis of 20 nm and a long axis of 100 nm) 40.0 g was added thereto and mixed. After water 30.0 g was added and stirred, a water dispersion of titanium oxide was obtained.

[Example 22]

KF-6013 (manufactured by Shin-Etsu Chemical Co., Ltd.: PEG-9 dimethicone, HLB: 10.0) 12.0 g was mixed with 1,3-BG 13.0 g and then silicone surface-treated, silica surface-treated titanium oxide microfine particles (STR-100W-LPT manufactured by Sakai Chemical Industry Co., Ltd.: spindle-shaped particles having a short axis of 20 nm and a long axis of 100 nm) 50.0 g was added thereto and mixed. After water 25.0 g was added and stirred, a water dispersion of titanium oxide was obtained.

[Example 23]

KF-6013 (manufactured by Shin-Etsu Chemical Co., Ltd.: PEG-9 dimethicone, HLB: 10.0) 5.0 g was mixed with 1,3-BG 15.0 g and then silicone surface-treated, silica surface-treated zinc oxide microfine particles (FINEX-50W-LP2 manufactured by Sakai Chemical Industry Co., Ltd.: average particle diameter of 20 nm) 60.0 g was added thereto and mixed. After water 20.0 g was added and stirred, a water dispersion of zinc oxide was obtained.

Sunscreen products of the following compositions were prepared by using these water dispersions.

[Example 24] (Sunscreen O/W cream)

Table 4

| | (Components) | (%) |
|---|---|---|
| 1 | Isotridecyl isononanoate | 14.8 |
| 2 | Ethylhexyl methoxycinnamate | 5.0 |
| 3 | Hexyl diethylaminohydroxybenzoylbenzoate | 2.0 |
| 4 | Three-dimentional crosslinked silicone (※1) | 4.0 |
| 5 | Behenyl alcohol | 1.0 |
| 6 | Sorbitan sesquiisostearate | 0.5 |
| 7 | Glyceryl stearate (SE) | 0.7 |
| 8 | Carbomer | 0.1 |
| 9 | Acrylate/alkyl acrylate (C10-C30) crosspolymer | 0.1 |
| 10 | Xanthan gum (2% aq) | 2.0 |
| 11 | PEG-20 sorbitan isostearate | 1.5 |
| 12 | Triethanolamine | 0.2 |
| 13 | 1,3-butylene glycol | 5.0 |
| 14 | Ethanol | 5.0 |
| 15 | Water dispersion of titanium oxide (example 21) | 25.0 |
| 16 | Purified water | 33.1 |

(※1)Three-dimentional crosslinked silicone: 9040 Silicone Elastomer Blend (manufactured by Dow Corning TORAY)

(Production method)

A: The components 1-7 are mixed uniformly and dissolved with heating.
B: The components 8-16 are mixed uniformly and heated.
C: A is added to B and the resultant is emulsified. The emulsion was cooled to obtain a sunscreen O/W cream.

The sunscreen O/W cream of the example 24 obtained as mentioned above is stable without change over time, and can spread smoothly on skin at coating, and does not cause a sticky feeling and creaking feeling after coating. Further, the sunscreen O/W cream has a high transparency, suppress whiteness, and is superior in maintaining the sunscreen effect.

[Example 25] (Sunscreen O/W cream)

Table 5

| | (Components) | (%) |
|---|---|---|
| 1 | Hydrogenated polyisobutene | 14.4 |
| 2 | Dispersion of zinc oxide fine particle/ethylhexyl palmitate (※2) | 15.4 |
| 3 | Behenyl alcohol | 1.0 |
| 4 | Sorbitan sesquiisostearate | 0.5 |
| 5 | Glyceryl stearate (SE) | 0.7 |
| 6 | (Sodium Acrylate/Sodium Acryloyldimethyl Taurate) Copolymer (※3) | 1.2 |
| 7 | Hydroxyethyl cellulose (2% aq) | 2.0 |
| 8 | PEG-20 sorbitan isostearate | 1.5 |
| 9 | 1,3-butylene glycol | 8.0 |
| 10 | Water dispersion of titanium oxide (example 22) | 25.0 |
| 11 | Purified water | 30.3 |

(※2)Dispersion of zinc oxide fine particle/ethylhexyl palmitate: dispersion obtained by mixing and dispersing FINEX-30S-LPT (manufactured by Sakai Chemical Industry Co., Ltd.: average particle diameter 35 nm) 65g, polyhydroxystearic acid 1.5g, and ethylhexyl palmitate 33.5g

(※3)(Sodium Acrylate/Sodium Acryloyldimethyl Taurate) Copolymer: SIMULGEL EG (manufactured by SEPPIC)

(Production method)

A: The components 1-5 are mixed and dissolved with heating.

B: The components 6-11 are mixed uniformly and heated.

C: A is added to B and the resultant is emulsified. The emulsion was cooled to obtain a sunscreen O/W cream.

The sunscreen O/W cream of the example 25 obtained as mentioned above is stable without change over time, and can spread smoothly on skin at coating, and does not cause a sticky feeling and creaking feeling after coating. Further, the sunscreen cream is an organic ultraviolet absorption agent-free sunscreen O/W cream which has a high transparency, suppress whiteness, and is superior in maintaining the sunscreen effect.

[Example 26] (Sunscreen O/W cream)

Table 6

| | (Components) | (%) |
|---|---|---|
| 1 | Hydrogenated polyisobutene | 18.6 |
| 2 | Three-dimentional crosslinked silicone (※1) | 4.5 |
| 3 | Behenyl alcohol | 0.7 |
| 4 | Sorbitan sesquiisostearate | 0.5 |
| 5 | Glyceryl stearate (SE) | 0.7 |
| 6 | (Sodium Acrylate/Sodium Acryloyldimethyl Taurate) Copolymer (※3) | 1.2 |
| 7 | Hydroxyethyl cellulose (2% aq) | 2.0 |
| 8 | PEG-20 sorbitan isostearate | 1.5 |
| 9 | 1,3-butylene glycol | 5.0 |
| 10 | Ethanol | 5.0 |
| 11 | Water dispersion of titanium oxide (example 22) | 15.0 |
| 12 | Water dispersion of zinc oxide (example 23) | 20.0 |

(continued)

| (Components) | (%) |
|---|---|
| 13   Purified water | 25.3 |

(Production method)

A: The components 1-5 are mixed and dissolved with heating.
B: The components 6-13 are mixed uniformly and heated.
C: A is added to B and the resultant is emulsified. The emulsion was cooled to obtain a sunscreen O/W cream.

The sunscreen O/W cream of the example 26 obtained as mentioned above is stable without change over time even though it has a system in which zinc oxide and titanium oxide are mixed and dispersed, and can spread smoothly on skin at coating, and does not cause a sticky feeling and creaking feeling after coating. Further, the sunscreen cream is an organic ultraviolet absorption agent-free sunscreen O/W cream which has a high transparency, suppress whiteness, and is superior in maintaining the sunscreen effect.

INDUSTRIAL APPLICABILITY

The water-based dispersion of the present disclosure can be suitably used in cosmetics.

**Claims**

1. A water-based dispersion which contains an inorganic powder microfine particle subjected to a hydrophobic organic surface treatment, a polyhydric alcohol, water, and a nonionic surfactant, wherein the nonionic surfactant is one or more compounds selected from the group consisting of a polyoxyalkylene fatty acid ester,
   a polyoxyalkylene sorbitan fatty acid ester, and an organopolysiloxane having a polyoxyalkylene group,
   the nonionic surfactant has an HLB value of 6 to 12, and
   wherein the nonionic surfactant is a compound which can be dissolved transparently or slightly muddy in 1,3-butylene glycol (20 wt% concentration, 35°C),
   and is insoluble or dispersed cloudy in water (20 wt% concentration, 35°C),
   and an amount of the inorganic powder microfine particle is 30 wt% or more,
   wherein dissolved transparently means that a haze value of the obtained mixture at an optical path length of 10 mm and 35°C is less than 10, and slightly muddy means that a haze value of the obtained mixture at an optical path length of 10 mm and 35°C is 10 or more, and the total light transmittance is 30% or more,
   and insoluble means that an unmelted residue is caused in the mixture, or a phase separation is occurred an hour later even if the mixture looks muddy, and dispersed cloudy means that a haze value of the obtained mixture at an optical path length of 10 mm and 35°C is 10 or more, and the total light transmittance is less than 30%.

2. The water-based dispersion according to claim 1,
   wherein the inorganic powder microfine particle is at least one selected from the group consisting of titanium oxide, zinc oxide, and cerium oxide.

3. The water-based dispersion according to claim 1 or 2,
   wherein a part surface or all surface of the inorganic powder microfine particle is covered by at least one selected from the group consisting of silica, aluminium hydroxide, alumina, and zirconia.

4. The water-based dispersion according to claim 1, 2, or 3,
   wherein the polyhydric alcohol is one or more polyhydric alcohol selected from the group consisting of propylene glycol, butylene glycol, pentane diol, dipropylene glycol, hexanediol, and heptanediol.

5. The water-based dispersion according to claim 1, 2, 3, or 4,
   wherein the nonionic surfactant is an organopolysiloxane nonionic surfactant.

6. Use of the water-based dispersion according to claim 1, 2, 3, 4, or 5, to obtain a cosmetic.

7. A method of preparing a cosmetic containing the water-based dispersion according to claim 1, 2, 3, 4 or 5, comprising a step of blending the water-based dispersion in the cosmetic.

**Patentansprüche**

1. Dispersion auf Wasserbasis, die mikrofeine anorganische Pulverpartikeln, die einer hydrophoben organischen Oberflächenbehandlung unterworfen werden, einen mehrwertigen Alkohol, Wasser und ein nichtionisches Tensid enthält, wobei das nichtionische Tensid eine oder mehrere Verbindungen ist, ausgewählt aus der Gruppe bestehend aus einem Polyoxyalkylen-Fettsäureester, einem Polyoxyalkylen-Sorbitan-Fettsäureester und einem Organopolysiloxan mit einer Polyoxyalkylen-Gruppe,
   wobei das nichtionische Tensid einen HLB-Wert von 6 bis 12 aufweist, und wobei das nichtionische Tensid eine Verbindung ist, die transparent oder leicht trüb in 1,3-Butylenglycol (20 Gew.-% Konzentration, 35 °C) aufgelöst werden kann und in Wasser (20 Gew.-% Konzentration, 35 °C) unlöslich oder trüb dispergiert ist und
   die Menge der mikrofeinen anorganischen Pulverpartikeln 30 Gew.-% oder mehr ist,
   wobei transparent aufgelöst bedeutet, dass der Trübungswert der erhaltenen Mischung bei einer optischen Weglänge von 10 mm und 35 °C kleiner als 10 ist, und leicht trüb bedeutet, dass der Trübungswert der erhaltenen Mischung bei einer optischen Weglänge von 10 mm und 35 °C 10 oder mehr ist und die Gesamt- Lichtdurchlässigkeit 30 % oder mehr ist, und unlöslich bedeutet, dass ein ungeschmolzener Rest in der Mischung entsteht oder eine Phasentrennung eine Stunde später auftritt, auch wenn die Mischung trüb aussieht, und trüb dispergiert bedeutet, dass der Trübungswert der erhaltenen Mischung bei einer optischen Weglänge von 10 mm und 35 °C 10 oder mehr ist, und die Gesamt-Lichtdurchlässigkeit kleiner als 30 % ist.

2. Dispersion auf Wasserbasis nach Anspruch 1, wobei
   die mikrofeinen anorganischen Pulverpartikeln ausgewählt sind aus der Gruppe bestehend aus Titaniumoxid, Zinkoxid und/oder Ceroxid.

3. Dispersion auf Wasserbasis nach Anspruch 1 oder 2, wobei
   ein Teil der Oberfläche oder die gesamte Oberfläche der mikrofeinen anorganischen Pulverpartikeln durch mindestens eine Verbindung ausgewählt aus der Gruppe bestehend aus Siliciumdioxid, Aluminiumhydroxid, Aluminiumoxid und Zirconiumdioxid bedeckt ist.

4. Dispersion auf Wasserbasis nach Anspruch 1, 2 oder 3, wobei
   der mehrwertige Alkohol einer oder mehrere mehrwertige Alkohole ist, ausgewählt aus der Gruppe bestehend aus Propylenglycol, Butylenglycol, Pentandiol, Dipropylenglycol, Hexandiol und Heptandiol.

5. Dispersion auf Wasserbasis nach Anspruch 1, 2, 3 oder 4,
   wobei das nichtionische Tensid ein nichtionisches Organopolysiloxan-Tensid ist.

6. Verwendung der Dispersion auf Wasserbasis nach Anspruch 1, 2, 3, 4 oder 5, um ein Kosmetikum zu erhalten.

7. Verfahren zur Herstellung eines Kosmetikums, das die Dispersion auf Wasserbasis nach Anspruch 1, 2, 3, 4 oder 5 enthält, umfassend einen Schritt des Vermischens der Dispersion auf Wasserbasis in dem Kosmetikum.

**Revendications**

1. Dispersion à base d'eau qui contient une particule microfine de poudre inorganique soumise à un traitement de surface organique hydrophobe, un alcool polyhydrique, de l'eau et un tensioactif non ionique, dans laquelle le tensioactif non ionique est un ou plusieurs composés choisis dans le groupe constitué par un ester d'acide gras polyoxyalkylène, un ester d'acide gras sorbitan polyoxyalkylène et un organopolysiloxane ayant un groupe polyoxyalkylène, le tensioactif non ionique a une valeur de HLB de 6 à 12, et dans laquelle le tensioactif non ionique est un composé qui peut être dissous de manière transparente ou légèrement boueuse dans du 1,3-butylène glycol (concentration de 20 % en poids, 35 °C),
   et est insoluble ou dispersé de manière nuageuse dans de l'eau (concentration de 20 % en poids, 35 °C),
   et une quantité de la particule microfine de poudre inorganique est de 30 % en poids ou plus,
   dans laquelle dissous de manière transparente signifie qu'une valeur de trouble du mélange obtenu à une longueur de chemin optique de 10 mm et à 35 °C est de moins de 10, et légèrement boueux signifie qu'une valeur de trouble du mélange obtenu à une longueur de chemin optique de 10 mm et à 35 °C est de 10 ou plus, et le facteur de transmission de lumière totale est de 30 % ou plus, et insoluble signifie qu'un résidu non fondu est entraîné dans le mélange, ou une séparation de phase est produite une heure plus tard même si le mélange semble boueux, et dispersé de manière nuageuse signifie qu'une valeur de trouble du mélange obtenu à une longueur de chemin optique de 10 mm et à 35 °C est de 10 ou plus, et le facteur de transmission de lumière totale est de moins de 30 %.

2. Dispersion à base d'eau selon la revendication 1,
dans laquelle la particule microfine de poudre inorganique est au moins une choisie dans le groupe constitué par l'oxyde de titane, l'oxyde de zinc et l'oxyde de cérium.

3. Dispersion à base d'eau selon la revendication 1 ou 2,
dans laquelle une surface de partie ou toute la surface de la particule microfine de poudre inorganique est recouverte par au moins un choisi dans le groupe constitué par la silice, l'hydroxyde d'aluminium, l'alumine et l'oxyde de zirconium.

4. Dispersion à base d'eau selon la revendication 1, 2 ou 3,
dans laquelle l'alcool polyhydrique est un ou plusieurs alcools polyhydriques choisis dans le groupe constitué par le propylène glycol, le butylène glycol, le pentane diol, le dipropylène glycol, l'hexanediol et l'heptanediol.

5. Dispersion à base d'eau selon la revendication 1, 2, 3 ou 4,
dans laquelle le tensioactif non ionique est un tensioactif non ionique organopolysiloxane.

6. Dispersion à base d'eau selon la revendication 1, 2, 3, 4 ou 5 pour obtenir un cosmétique.

7. Procédé de préparation d'un cosmétique contenant la dispersion à base d'eau selon la revendication 1, 2, 3, 4 ou 5, comprenant une étape de mélange de la dispersion à base d'eau dans le cosmétique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2852487 B

- WO 2013018827 A